Europäisches Patentamt

European Patent Office

Office européen des brevets

(11) Publication number: **0 126 338**
**B1**

(12) **EUROPEAN PATENT SPECIFICATION**

(45) Date of publication of patent specification: **23.01.91**

(21) Application number: **84104774.9**

(22) Date of filing: **27.04.84**

(51) Int. Cl.⁵: **C 12 N 15/00, C 12 P 21/00 //
C12R1/19**

(54) Novel process for expressing a foreign gene.

(30) Priority: **28.04.83 JP 75720/83**

(43) Date of publication of application:
**28.11.84 Bulletin 84/48**

(45) Publication of the grant of the patent:
**23.01.91 Bulletin 91/04**

(84) Designated Contracting States:
**DE FR GB**

(56) References cited:
**EP-A-0 064 681**
**EP-A-0 083 069**

**DNA, vol.1, no.2 1982, New york, USA. H M
SHEPARD et al. "Increased Synthesis in E. coli
Fibroblast and Leucocyte Interferons Through
Alterations in Ribosome Binding Sites"**

**NUCLEIC ACIDS RESEARCH, vol. 9, no.24,
December 21 1981, London GB. C YANOFSKY et
al. "The complete nucleotide sequence of the
tryptophan operon of Escherichia coli" pages
6647-6668**

(73) Proprietor: **KYOWA HAKKO KOGYO CO., LTD.
Ohtemachi Bldg., 6-1 Ohtemachi I-chome
Chiyoda-ku Tokyo 100 (JP)**

(72) Inventor: **Nishi, Tatsunari
5-19-24 Okura
Setagaya-ku Tokyo (JP)**
Inventor: **Saito, Akiko
Shiroyamaen 201 1522, Honmachida
Machida-shi Tokyo (JP)**
Inventor: **Itoh, Seiga
218-14, Aihara
Sagamihara-shi Kanagawa-ken (JP)**

(74) Representative: **Casalonga, Axel et al
BUREAU D.A. CASALONGA - JOSSE
Morassistrasse 8
D-8000 München 5 (DE)**

(56) References cited:
**CELL, vol. 18, no.4, December 1979, Cambridge,
Mass. K NAKAMURA et al. "DNA Sequence of
the Gene for the Outer Membrane Lipoprotein
of E. coli; an extremely AT-Rich Promoter"
pages 1109-1117**

Courier Press, Leamington Spa, England.

## Description

### Background of the Invention

This invention relates to a novel process for expressing a foreign gene. More particularly, the present invention relates to a process for inserting a foreign gene into an operon of microorganism, whereby the operon is reconstructed to form polycistron and to express the foreign gene efficiently.

With recent development of recombinant DNA technique, mass production of heteroprotein by utilizing a microorganism, particularly Escherichia coli has become possible. An increase in expression efficiency of heteroprotein genes in host cells is an important subject for the development of recombinant DNA technique.

To increase the expression efficiency, a method for increasing a transcription efficiency by using promoters of lac system and trp system as promoters which are the necessary genetic information for transcription initiation, a method for increasing a translation efficiency by using a recombinant with a changed distance and a changed DNA sequence between the ribosome binding site (SD sequence) and translation initiation codon (mainly ATG) [H. M. Shepard et al: DNA 1, 125 (1982)], etc. have been developed.

It is also known that, when a gene of eucaryote origin is linked to the initial region of structural gene of procaryote having a high translation initiation efficiency and is expressed in the form of fused protein, the gene can be expressed with high efficiency. However, in the case of physiologically active peptides destined to administration to human bodies, such as human growth hormone, interferon, etc., the protein in the fused form with the protein of procaryote origin has problems such as side effects, etc., and thus development of a process for expressing a foreign gene to form protein free from the protein of procaryote origin has been desired.

The present inventors have made extensive studies directed to efficient expression of a foreign gene and have found that a foreign gene can be efficiently expressed by a plasmid prepared by reconstructing an operon by inserting a foreign gene into an operon of microorganism to form polycistron and inserting the operon into a plasmid.

### Summary of the Invention

The present invention provides a process for expressing a foreign gene by using an operon reconstructed by recombining an operon or a part of the operon with a foreign gene to form polycistron.

The operon includes operons of procaryote origin, for example, polycistron-formed operons such as tryptophan (trp) operon of Escherichia coli [C. Yanofsky et al: Nucleic Acids Research 9, 6647 (1981)], threonine operon [Pascle Cossart et al: Nucleic Acids Research 9, 339 (1981))], φ ×174 phage operon [F. Sanger et al: Nature 265, 687 (1977), J. Mol. Biol. 125, 225 (1978)], λphage operon [F. Sanger et al: J. Mol. Biol. 162, 729 (1982)].

It is known that there is an SD sequence necessary for translation initiation of the cistrons upstream from the individual cistrons in these polycistrons, and the second and subsequent cistrons have, near the SD sequence, a termination codon of the cistron which exists right upstream, or the translation initiation codon and the SD sequence are overlapped, and thus in reconstruction of operon, a foreign gene is inserted into the operon to form a base sequence which can satisfy these conditions for polycistron.

It is preferable that the operon for use in the present invention has a very active promoter and that a gene with a good translation initiation efficiency is located at the first cistron.

DNA sequence around the translation initiation codon and the SD sequence has been statistically analyzed, and an optimum structure for translation initiation [Nucleic Acids Research 8, 3895 (1980)], rules necessary or suitable for translation initiation [Nucleic Acids Research 10, 2971 (1982)], etc. have been reported. In the reports it is mentioned that one of the most suitable characteristic structures for translation initiation is that the DNA base sequence around the translation initiation codon is rich in adenine and thymine bases. For example, the DNA sequence around the translation initiation codon in the gene coding lipoprotein (lpp) [Cell 18, 1109 (1979)], alkaline phosphatase (phoA) [Nucleic Acids Research 9, 5671 (1981)], ribosome protein, etc. is rich in adenine and thymine bases. Thus, it is necessary to adjust not only the structure between the SD sequence and the translation initiation codon, but also the structure around the translation initiation codon including the base sequence downstream from the translation initiation codon to form the structure necessary for efficient translation initiation.

However, when a foreign gene is linked downstream from the promoter and SD sequence in Escherichia coli to express the foreign gene, it is impossible to change the DNA base sequence downstream from the translation initiation codon in the foreign gene to a structure suitable for translation initiation of Escherichia coli, and thus the protein-synthesizing capacity of Escherichia coli cannot be fully utilized.

The present process is useful for fully utilizing the protein-synthesizing capacity of Escherichia coli. That is, a structural gene with a high translation initiation efficiency must be located downstream from an active promoter of Escherichia coli, and the desired foreign genes are located downstream as the second and subsequent cistrons to form polycistron. When an operon is reconstructed in this manner, the ribosome molecule initiates translation efficiently at first from the first cistron, using the mRNA transcribed from the said active promoter site as a template, and subsequently the same ribosome molecule translates the second and subsequent cistrons and synthesizes protein. As a result, the second and subsequent cistrons are translated with the same efficiency as that for the first cistron to synthesize protein by adjusting the linking condition with the preceding cistron, and thus it is possible to express the foreign gene by fully utilizing the protein-synthesizing capacity of *Escherichia coli*.

Brief Description of the Drawings

Fig. 1 shows a restriction enzyme map of λwild strain DNA, λcI857trpED10 phage DNA and λcI857trpED10Δ LE1417 phage DNA.

Fig. 2 shows a flowchart for the construction of pKYP—1 and pKYP—2.

Fig. 3 shows a flowchart for the construction of pKYP—8 and pKYP—9.

Fig. 4 shows a flowchart for the construction of pKYP—108 and pKYP—109.

Fig. 5 shows the DNA sequence of the trp operon region in pKYP—108 and pKYP—109.

Fig. 6 shows a flowchart for the construction of pFN—4.

Fig. 7 shows the DNA sequence of the trpE gene region and its downstream region in pFN—4.

Fig. 8 shows a flowchart for the construction of pOA—15 and pOAA—101, wherein Ptrp is a trp promoter, $P_{lpp}$ is an lpp promoter, $P_{lac}$ is an lac promoter, Xb is XbaI, E is EcoRI, H is HindIII, B is BamHI, and S is a SalI.

Fig. 9 is a flowchart for the construction of pKYP—5.

Fig. 10 is a flowchart for the construction of pKYP—10.

Fig. 11 is a flowchart for the construction of pKYP—100.

Fig. 12 is a flowchart for the construction of pLE—3.

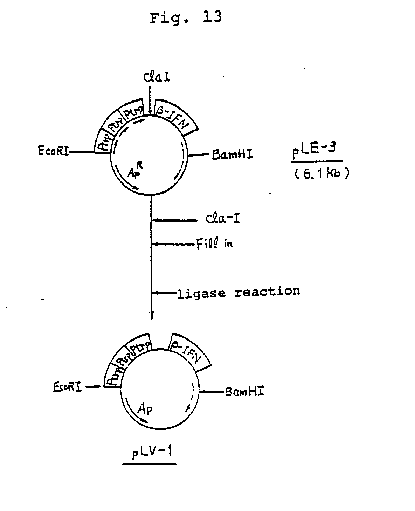

Fig. 13 is a flowchart for the construction of pLV—1.

Fig. 14 is a flowchart for the construction of pNJE—1.

Detailed Description of the Invention

As a specific example of the present process for the expression, a procedure utilizing a trp operon with high transcription and translation efficiencies, whose transcription and translation mechanisms have been clarified relatively in detail, will be described below:

The trp operon of *Escherichia coli* consists of 5 structural genes coding for enzymes which take part in biosynthesis of tryptophan from chorismic acid (trpE, trpD, trpC, trpB, and trpA) and regions regulating expression of these genes (trpP, trpO, trpL and trpa). The five structural genes of the trp operon are transcribed as one polycistronic mRNA having a length of about 7,000 bases, and transcription initiation is regulated by the interaction between the trp repressor (product of trpR gene) and the operator site (trpO). The region between the transcription initiation site existing in the promoter (trpP)-operator (trpO) region and the trpE gene is called a leader region, which includes the trpL gene coding for leader peptide and attenuator site (trpa). If the amount of tryptophanyl-tRNA in the cell is large, the transcription terminates at the attenuator site, and no more transcription of the 5 structural genes following the leader region occurs, whereas if the amount of tryptophanyl-tRNA is small, transcription of the 5 structural genes occurs without termination at the attenuator site.

Transcription of trp operon having such a structure is controlled by repression and attenuation in vivo. That is, repression reduces the transcription efficiency to 1/70 and attenuation to 1/8—1/10. When the cells of *Escherichia coli* are deficient in tryptophan, the transcription is free from repression and attenuation, and transcription efficiency increases about 600-fold at a maximum, as compared with the case where the cells are abundant in tryptophan.

When an operon is reconstructed by inserting a foreign gene downstream from the first of the five structural genes so as to form polycistron, it is possible to express the foreign gene with the same transcription and translation efficiencies as those of trp operon.

As a source of DNA containing trp operon, plasmids pKYP—1, pKYP—2, pKYP—8, pKYP—9, pKYP—108 and pKYP—109 and their derivatives can be used. These plasmids can be prepared in the manner disclosed in Examples.

The said DNA is cut (digested) at recognition sites of appropriate restriction enzymes existing in the five structural genes of a trp operon, for example, HindIII site, ClaI site, BalI site, BglI site, BglII site, EcoRV site, HincII site, NruI site,, PvuII site, SstII site, etc. with these restriction enzymes.

Digestion of DNA is carried out usually by subjecting 0.1—100 µg of DNA to digestion reaction in a solution consisting of 2—200 mM, preferably 10—40 mM Tris-HCl (pH 6.0—9.5, preferably 7.0—8.0), 1—150 mM NaCl and 2—20 mM, preferably 5—10 mM $MgCl_2$ in the presence of 0.3—300 units, preferably 1—3 units of the enzyme per 1 µg of DNA at 18—42°C, preferably 32—38°C for 15 minutes to 24 hours. The reaction can be terminated usually by heating at 55—75°C, preferably 63—70°C, for 5—30 minutes, but a procedure for inactivating the restriction enzyme by a reagent such as phenol or diethylpyrrocarbonate can also be used.

To the terminal of the resulting DNA fragment is ligated a DNA fragment having, at an appropriate position upstream from the foreign gene, the SD sequence necessary for translation of the foreign gene to be expressed.

As the foreign gene to be expressed, genes of eucaryote, for example, genes coding for physiologically active peptides such as inteferon, insulin, growth hormone, etc. can be mentioned. Preferably, β-IFN can be used.

The SD sequence to be added is the one derived from a gene, such as lacZ gene, trpL gene, lpp gene or phoA gene.

The appropriate position upstream from the gene is a position at which the distance between the SD sequence and the translation initiation codon is a distance which enables efficient translation initiation, usually at 3—20 bases upstream from the gene.

Ligation of the DNA fragments is carried out by subjecting both DNA fragments to reaction in a solution consisting of 2—200 mM, preferably 10—70 mM Tris-HCl (pH 6.0—9.5, preferably 7.0—8.0), 2—20 mM, preferably 5—10 mM MgCl$_2$, 0.1—10 mM, preferably 0.5—2 mM ATP and 1—50 mM, preferably 5—10 mM dithiothreitol in the presence of 0.1—10 units of T4DNA ligase at 1—37°C, preferably 3—20°C for 15 minutes to 72 hours, preferably 2—20 hours. The reaction is terminated in the same manner as in the above digestion.

In ligating DNA fragments, it is necessary that the reading frame for translation of a gene of trp operon origin existing before the foreign gene is terminated at a position which enables translation of the foreign gene, that is, around the SD sequence added upstream from the foreign gene or the near translation initiation codon of the foreign gene.

The thus ligated DNA fragments are inserted into an appropriate plasmid to form a recombinant plasmid, and the phenotype of the foreign gene can be expressed by inserting the recombinant plasmid into an appropriate host microorganism and culturing the microorganism.

As an appropriate plasmid, pBR322, pBR325, pBR327, pBR328, pBR329, pACYC177, pACY184, pGA22, etc. can be mentioned.

As an appropriate host microorganism, the wild strain of *Escherichia coli* K12 and its mutants, for example, strain HB101, strain C600, etc. can be mentioned.

Construction of a recombinant plasmid and culturing of transformants can be carried out according to the procedures disclosed in Japanese Patent Application 213193/81 and Nucleic Acids Research *8*, 4057 (1980).

In the foregoing, the present invention has been described referring to the trp operon of *Escherichia coli*, but the present process can also be carried out with operons of *Escherichia coli* other than trp operon, for example, lipoprotein operon and threonine operon, or furthermore with operons of procaryote other than *Escherichia coli*.

Certain specific embodiments of the invention are described in the following representative examples.

Example 1

Construction of plasmid vectors pKYP—1 and pKYP—2 having a trp operon region from trp promoter to a point in trpD gene:

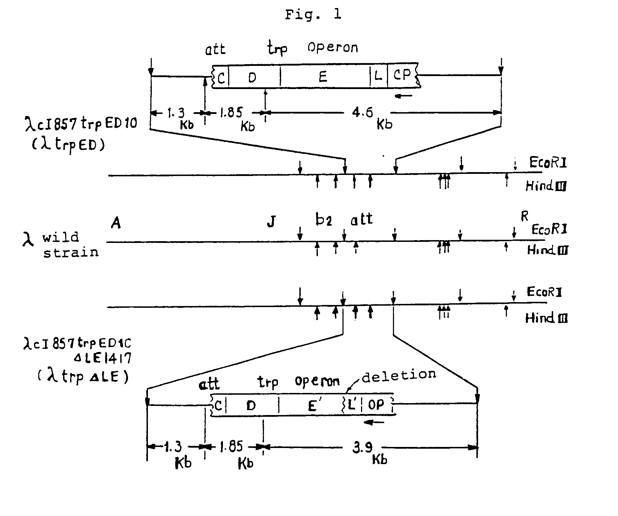

Strain JA194 (λtrpED) obtained by lysogenizing λcl857trpED10 [see Fig. 1, hereinafter referred to as λtrpED; G. F. Miazzari et al: J. Bacteriol. *133*, 1457 (1978)] as λtrp phage in *Escherichia coli* strain JA194 [F⁻λ⁻rk⁻mk⁻ΔtrpE5, leu-6; J. Carbon et al: Recombinant Molecules, p.355 (1977) Raven Press] [J. H. Miller: Experiments in Molecular Genetics p.274 (1972)] was cultured in an L broth (10 g/l bactotryptone, 5 g/l yeast extract and 5 g/l NaCl, pH 7.2) at 42°C for 30 minutes to induce a λtrpED phage and prepare a phage lysate.

From this phage lysate was purified λtrpED phage according to a cesium chloride equilibrium density gradient centrifuge method [Yamakawa et al: "Kajusan no Kagaku I" p.54—61 (1974) Tokyo Kogaku Dojin-sha]. Then, from this λtrpED phage was purified DNA by phenol treatment and chloroform treatment according to the method of Yamakawa et al (ibid, p.62—65).

Separately, DNA was purified from λcl 875trpE-D10ΔLE417 phage having a a deletion mutation of about 720 bp in the trpL and trpE gene region of λcl857trpED10 phage [see Fig. 1, hereinafter referred to as "λtrpEDΔLE"; G. F. Miazzari et al: J. Bacteriol. *133*, 1457 (1978)].

Cloning of the trp operon from λtrpED phage DNA was carried out as follows:

In 30 μl of a reaction solution containing 20 mM Tris-HCl (pH 7.5), 75 mM NaCl, 10 mM MgCl$_2$ and 5 mM dithiothreitol, 8 μg of λtrpED phage DNA was subjected to digestion reaction in the presence of 16 units of EcoRl (made by Takara Shuzo Co., hereinafter the same product will be used under this designation) and 16 units of HindIII (made by Takara Shuzo Co., hereinafter the same product will be used under this designation) at 37°C for 2 hours.

Likewise, λtrpEDΔLE phage DNA was subjected to digestion reaction.

Separately, 1 μg of plasmid pBR325DNA [F. Bolivar et al: Gene *4*, 121 (1978)] was likewise subjected to digestion reaction in the same reaction solution (30 μl) in the presence of 2 units of EcoRl and 2 units of HindIII.

The digestion reaction solutions were heated at 65°C for 5 minutes to terminate the reaction, and 15 μl each of digested DNA solution of phage DNA and digested DNA solution of plasmid were mixed and subjected to ligation reaction in the presence of 0.5 mM (final concentration) ATP and 5 units of T4DNA ligase (made by New England Biolobs; hereinafter the same product will be used under the same designation) at 4°C for 18 hours.

With the thus obtained recombinant plasmid DNA, *Escherichia coli* C600SF8 strain [Cameron et al: Proc. Natl. Acad. Sci, *72*, 3416 (1975)] was transformed according to the known procedure [S. N. Cohen et al: Proc. Natl. Acad. Sci, *69*, 2110 (1972)] to obtain a transformer having ampicillin resistance (Ap$^R$), tetracyclin resistance (Tc$^R$) and chloramphenicol sensitivity (Cm$^S$).

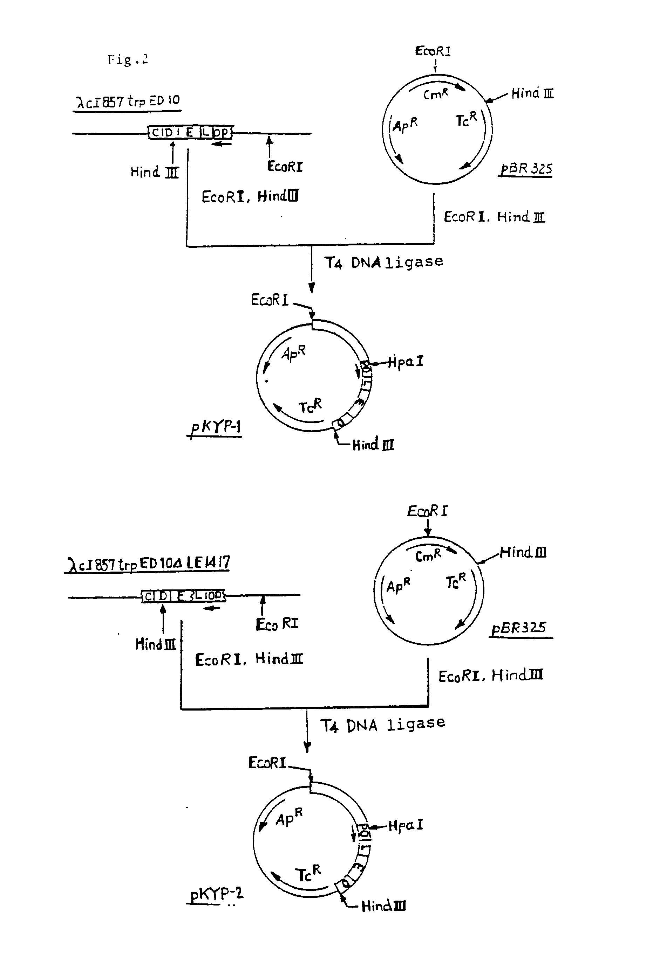

From the transformants were isolated and purified plasmid DNAs according to the known procedure [H. C. Birnboim et al: Nucleic Acids Res. *7*, 1513 (1979)], and the DNAs were digested with restriction enzymes EcoRl, Hpal, HindIII, Pstl (all of these products were made by Takara Shuzo Co.), etc. to make structural analysis of the plasmids. As a result, it was found that pKYP—1 in which the trp operon of λtrpED origin from the trp promoter to a point in the trpD gene was cloned was constructed (see Fig. 2).

Likewise, λtrpEDΔLE phage DNA was subjected to digestion, ligation and transformation to construct pKYP—2, in which the trp operon of λtrpE-DΔLE origin was cloned (see Fig. 2).

The *Escherichia coli* strains containing pKYP—1 and pKYP—2, respectively, have been deposited with the Fermentation Research Institute, Agency of Industrial Science and Technology, as *Escherichia coli* IKYP—1, FERM P—6962 (FERM BP—519) and IKYP—2, FERM P—6963 (FERM BP—520), respectively.

These plasmid vectors pKYP—1 and pKYP—2 can be applied to efficient expression of foreign genes by reconstructing the operon by ligating the foreign genes downstream from BglII site in the trpE gene or HindIII site in the trpD gene so as to form polycistron.

### Example 2

Construction of plasmid vectors pKYP—8 and pKYP—9 having a trp operon region from trp promoter to a point in trpE gene:

About 30 μg of DNA or pKYP—1 obtained in Example 1 was digested with about 1.5 units of TaqI (made by New England Biolabs) at 45°C for 60 minutes in 100 μl of a buffer solution containing 10 mM Tris-HCl (pH 7.5), 100 mM NaCl, 7 mM MgCl₂ and 6 mM 2-mercaptoethanol (hereinafter the buffer solution will be referred to as "Y—100 buffer solution"), whereby one molecule of pKYP—1 plasmid DNA was cut by TaqI at one location on an average. The digestion reaction was terminated by phenol extraction, and the reaction product was subjected to chloroform extraction and ethanol precipitation. Precipitated DNA fragments were dissolved in 100 μl of Y—100 buffer solution, and subjected to reaction in the presence of 80 units of EcoRI at 37°C for 2 hours. The reaction was terminated by heat treatment at 65°C for 10 minutes, and two DNA fragments of about 2.8 kb (kilobases) and about 2.9 kb, each containing the trp promoter region and a part of the trpE gene were purified according to a low gelling temperature agarose gel electrophoresis method [Lars Wieslander: Analyt. Biochem. 98, 305 (1979); this method will be hereinafter used for purification of DNA by agarose electrophoresis].

Separately, 5 μg of pBR325 was subjected to reaction in 30 μl of a digestion reaction solution containing 10 mM tris-HCl (pH 8.0), 10 mM MgCl₂ and 6 mM 2-mercaptoethanol in the presence of 10 units of ClaI (made by Boehringer-Mannheim, hereinafter the same product will be used under the same designation) at 37°C for 2 hours. Then, the reaction was terminated by heat treatment at 65°C for 10 minutes, and then 2M NaCl was added to the reaction mixture to make the NaCl concentration 100 mM. The reaction mixture was further subjected to reaction in the presence of 8 units of EcoRI at 37°C for 2 hours.

Then, about 0.15 μg of pBR325 DNA digested with ClaI and EcoRI as above and about 0.2 μg each of the above two DNA fragments of about 2.8 kb and about 2.9 kb, respectively, containing the trp promoter region and a part of the trpE gene were subjected to ligation reaction in 20 μl

of a buffer solution containing 20 mM Tris-HCl (pH 7.6), 10 mM MgCl₂, 10 mM dithiothreitol and 0.5 mM ATP (hereinafter the buffer solution will be referred to as "ligase buffer solution") in the presence of 2 units of T4DNA ligase at 4°C for 18 hours.

With the thus obtained recombinant plasmid DNA, *Escherichia coli* C600SF8 strain was transformed in the same manner as above to obtain transformants of Ap^R Tc^R Cm^S.

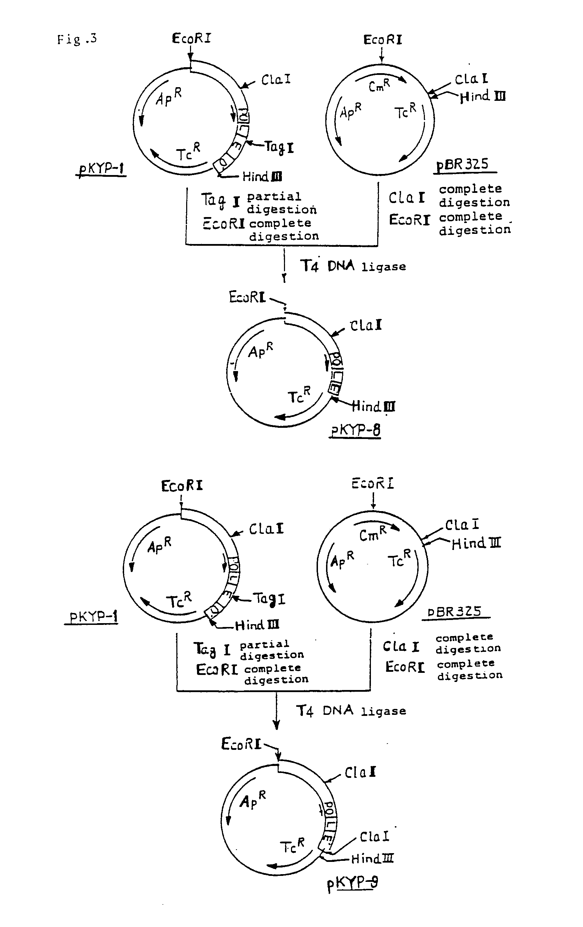

Plasmid DNAs were isolated and purified from the transformants in the same manner as above, and the DNAs were digested with 5 kinds of restriction enzymes HpaI, PstI, EcoRI, HindIII and ClaI to make structural analysis of the plasmids. It was found that pKYP—8 wherein the fragment of abot 2.8 kb containing the trp operon was inserted between EcoRI site and ClaI site of pBR325 (see Fig. 3) and pKYP—9 wherein the fragment of about 2.9 kb containing the trp operon was inserted (see Fig. 3) were constructed.

The base sequence around the HindIII site of pKYP—8 and pKYP—9 was examined according to the method of Maxam-Gilbert [A. M. Maxam et al: Proc. Natl. Acad. Sci. 74, 560 (1977)], and it was found that pKYP—8 was cloned with respect to the part from the N terminal to the 8th amino acid residue of the trpE protein and pKYP—9 was cloned with respect to the part up to the 43rd amino acid residue.

The *Escherichia coli* strains containing pKYP—8 and pKYP—9, respectively, have been deposited with the Fermentation Research Institute as *Escherichia coli* IKYP—8, FERM P—6964 (FERM BP—521) and IKYP—9, FERM P—6965 (FERM BP—522), respectively.

These plasmid vectors pKYP—8 and pKYP—9 can be applied to efficient expression of foreign genes by reconstructing the operon by ligating the foreign genes downstream from HindIII site, etc. in the trp gene so as to form polycistron.

### Example 3

Constructive of derivatives pKYP—108 and pKYP—109 from plasmid vectors pKYP—8 and pKYP—9, respectively:

To make the plasmid vectors pKYP—8 and pKYP—9 constructed in Example 2 more readily applicable, derivatives pKYP—108 and pKYP—109 freed from the excess DNA region located upstream from the trp promoter by deletion were obtained in the following manner.

First of all, 16 units of BamHI (made by Takara Shuzo Co., hereinafter the same product will be used under the same designation) and 16 units of HpaI were added to 8 μg of plasmid pKYP—8 DNA, and the mixture was subjected to reaction in 50 μl of a Y—100 buffer solution at 37°C for 2 hours. The reaction was terminated by heat treatment at 65°C for 10 minutes, and the smaller plasmid DNA fragment (about 550 bp) [about 650 bp in the case of pKYP—9] was separated and purified by low gelling temperature agarose gel electrophoresis.

Separately, 8 units of BamHI and 8 units of HpaI

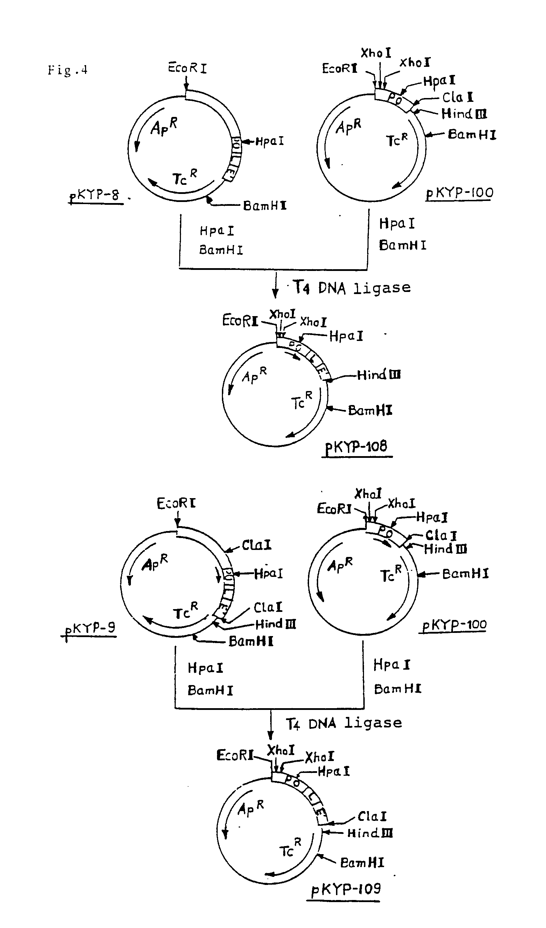

were added to about 4 µg of DNA of plasmid vector pKYP—100 having a trp portable promoter (see Japanese Patent Application No. 177192/82, Reference Example 2) [since this vector has two inserted XhoI linkers (CCTCGAGG) downstream from EcoRI site, a DNA fragment having a trp promoter can be ultimately cut off with a size of 124 bp with EcoRI and HindIII; see Fig. 4], and the mixture was subjected to digestion reaction in 30 µl of a Y—100 buffer solution at 37°C for 2 hours. Then, the reaction was terminated by heat treatment at 65°C for 10 minutes, and the larger plasmid DNA fragment (about 4.1 kb) was purified by low gelling temperature agarose gel electrophoresis.

About 0.1 µg of the thus obtained DNA fragment of pKYP—8 origin (about 550 bp) and about 0.15 µg of the thus obtained DNA fragment of pKYP—100 origin (about 4.1 kb) were admixed with 2 units of T4DNA ligase in 20 µl of a ligase buffer solution, and the mixture was subjected to ligation reaction at 4°C for 18 hours.

With the thus obtained recombinant plasmid DNA, *Escherichia coli* HB101 strain [Bolivar et al: Gene *2*, 75 (1977)] was subjected to transformation to obtain transformants of Ap$^R$Tc$^R$.

Plasmid DNAs were isolated and purified from the transformants in the same manner as above, and structural analysis of plasmids was carried out by digesting the DNAs with restriction enzymes EcoRI, HpaI, HindIII, BamHI and XhoI. As a result, it was found that pKYP—108 having the structure wherein the DNA fragment of pKYP—8 origin (288 bp) containing the trp promoter region, the trpL gene and a part of the trpE gene could be cut off with EcoRI and HindIII (see Fig. 4) was constructed.

Likewise, pKYP—109 having the structure wherein the DNA fragment of pKYP—9 origin (393 bp) containing the trp promoter region, the trpL gene and a part of the trpE gene could be cut off with EcoRI and HindIII (see Fig. 4) was constructed.

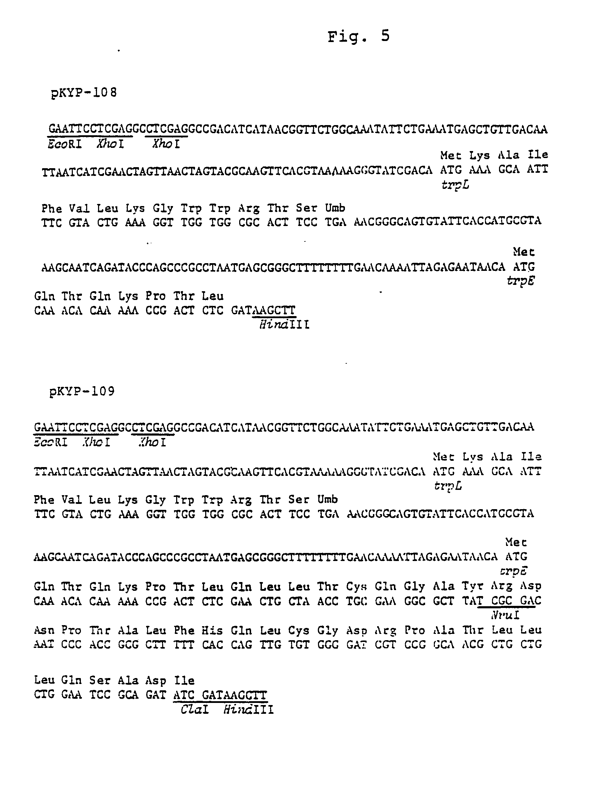

Base sequences of the trp region of pKYP—108 and pKYP—109 were examined according to the method of Maxam-Gilbert, and the results are shown in Fig. 5.

*Escherichia coli* strains containing pKYP—108 and pKYP—109, respectively, have been deposited with the Fermentation Research Institute as *Escherichia coli* IKYP—108, FERM P—6966 (FERM BP—523) and IKYP—109, FERM P—6967 (FERM BP—524), respectively.

These plasmid vectors pKYP—108 and pKYP—109 can be applied to efficient expression of foreign genes by reconstructing the operon by ligating the foreign genes downstream from the HindIII site, etc. so as to form polycistron.

### Example 4

Production of β-IFN by utilizing trp operon:

Plasmid vector pKYP—109 constructed in Example 3 and recombinant plasmid pLV—1 having a β-IFN gene were ligated at the NruI site existing in the trpE gene of pKYP—109 and the HpaI site existing in the trp promoter of pLV—1. The plasmid pLV—1 is disclosed in Japanese Patent Application No. 213193/81 and *Escherichia coli* ILV—1 containing pLV—1 has been deposited with the American Type Culture Collection under ATCC39025 (see Reference Example 4). By the ligation, an operon was reconstructed so that the gene coding for a polypeptide consisting of 30 amino acids wherein 12 excess amino acids were added to 18 amino acids at the N terminal of trpE protein can form a polycistron, and a system for expressing β-IFN was constructed in the following manner.

At first, 12 units of NruI (made by New England Biolabs, hereinafter the same product will be used under the same designation) and 16 units of EcoRI were added to 8 µg of pKYP—109 DNA, and the mixture was subjected to reaction in 50 µl of a Y—100 buffer solution at 37°C for 2 hours. Then, the reaction was terminated by heat treatment at 65°C for 10 minutes, and an EcoRI-NruI DNA fragment of 313 bp containing the trp promoter region was separated and purified by low gelling temperature agarose gel electrophoresis.

Separately, plasmid pLV—1 was isolated from *Escherichia coli* ATCC39025 according to the conventional method (M. Kahn et al: Methods in Enzymology *68*, 268 (1979)], and 8 units of HpaI and 8 units of EcoRI were added to 4 µg of pLV—1 DNA. The mixture was subjected to digestion reaction in 50 µl of a Y—100 buffer solution at 37°C for 2 hours. Then, the reaction was terminated by heat treatment at 65°C for 10 minutes, and EcoRI-HpaI DNA fragment of about 5.2 Kb containing the β-IFN gene was separated and purified by low gelling temperature agarose gel electrophoresis.

About 50 ng of the thus obtained EcoRI-NruI DNA fragment of pKYP—109 origin and about 150 ng of the thus obtained EcoRI-HpaI DNA fragment of pLV—1 origin were subjected to ligation reaction in 20 µl of a ligase buffer solution in the presence of 2 units of T4DNA ligase at 4°C for 18 hours.

With the thus obtained recombinant plasmid DNA, *Escherichia coli* HB—101 strain was transformed to obtain transformants of Ap$^R$Tc$^S$.

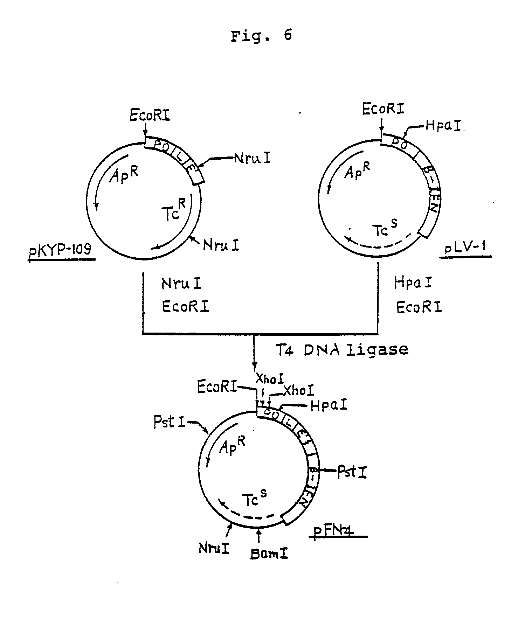

Plasmid DNAs were isolated and purified from the transformants in the same manner as above, and structural analysis of plasmids was carried out by digesting the DNAs with restriction enzymes EcoRI, XhoI, HpaI, NruI and BamHI. As a result, it was found that the desired plasmid pFN—4 having a structure shown in Fig. 6 was constructed.

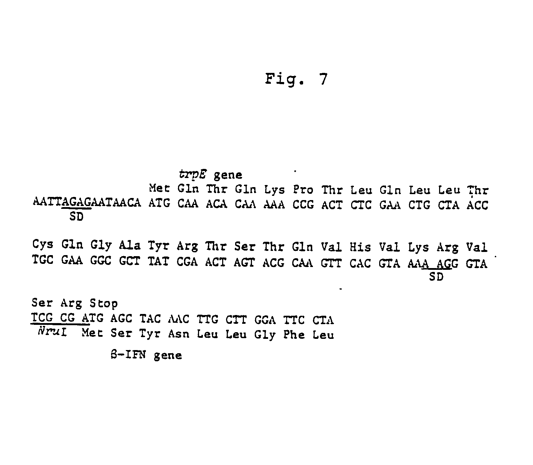

The recombinant plasmid pFN—4 was constructed by ligating the EcoRI-NruI DNA fragment of pKYP-109 origin to the EcoRI-HpaI DNA fragment of pLV—1 origin, which was examined according to the method of Maxam-Gilbert. That is, DNA base sequence at the ligating part of NruI site and HpaI site of pFN—4 was examined in the upstream direction from the NruI site just before the β-IFN gene of pLV—1 origin, and as a result, it was found that the ligating part had an expected DNA base sequence, and the β-IFN gene was located downstream from the gene coding for a peptide consisting of 30 amino acids wherein 12 excess amino acids were added to 18 amino acids at the N terminal of the trpE protein so as to form a polycistron (see Fig. 7).

Furthermore, as shown in Fig. 7, there is a SD sequence (AAGG) of trpL gene origin located 8 bases apart upstream from the translation initiation codon of β-IFN gene, which can reinitiate protein synthesis of ribosome. The termination codon of the gene coding for a polypeptide consisting of 30 amino acids existing upstream from β-IFN gene overlaps with the translation initiation codon of β-IFN gene.

*Escherichia coli* strain containing pIFN—4 has been deposited with the Fermentation Research Institute as *Escherichia coli* IFN—4, FERM P—6968 (FERM BP—525).

Production of β-IFN with the said strain was carried out in the following manner:

The strain was cultured in an LG broth (solution of 10 g of tryptone, 5 g of yeast extract, 5 g of NaCl, and 1 g of glucose in 1 l of water adjusted to pH 7.2 with NaOH) at 37°C for 18 hours. Then, 0.2 ml of the culture liquor was inoculated into 10 ml of an MCG medium [0.6% $Na_2HPO_4$, 0.3% $KH_2PO_4$, 0.5% NaCl, 0.1% $NH_4Cl$, 0.5% glucose, 0.5% casamino acid, 1 mM $MgSO_4$, and 4 μg/ml thiamine hydrochloride, pH 7.2] and cultured at 30°C for 4—8 hours. Then, 10 μg/ml indoleacrylic acid which is an inducer of tryptophan gene was added thereto, and culturing was further continued for 5—12 hours.

Then, the culture liquor was centrifuged at 8,000 rpm for 10 minutes to collect cells, and the cells were washed with 30 mM NaCl and 30 mM Tris-HCl buffer solution (pH 7.5). The washed cells were suspended in 1 ml of the said buffer solution, and 200 μg of lysozyme and 5 μl of 0.25 M EDTA (ethylenediaminetetraacetic acid) were added. The suspension was left standing at 0°C for 30 minutes, and subjected to three cycles of freezing and thawing to break the cells.

Then, the suspension was centrifuged at 15,000 rpm for 30 minutes to obtain a supernatant. The amount of interferon in the supernatant was determined according to the method of Armstrong [J. A. Armstrong: Appl. Microbiol. *21*, 723—725 (1971)], wherein Vesicular Stomatitis Virus was used as a challenge virus, and Wish cell of human amnion cell origin was used as an animal cell. As a result, it was found that the strain produced $5 \times 10^7$ units/l interferon.

### Example 5

Construction of recombinant plasmid pOA—15 in which β-IFN gene was inserted in expression vector pIN—II—A2:

First of all, 2 μg of pIN—II—A2 (same as pKEN039 in Japanese Published Unexamined Patent Application No. 140800/82) was dissolved in 30 μl of a Y—100 buffer solution, and 4 units of EcoRI and 4 units of BamHI were added thereto. The mixture was subjected to digestion reaction at 37°C for 2 hours, and then the reaction was terminated by heat treatment at 65°C for 5 minutes. Then, about 1.2 μg of DNA fragment of about 5 Kb containing lipoprotein (1pp) promoter·lac promoter was separated and purified by low gelling temperature agarose gel electrophoresis.

Separately, 4 μg of plasmid pNJE—1 prepared according to the method of Reference Example 5 was dissolved in 30 μl of a Y—100 buffer solution, and 6 units of EcoRI and 6 units of BamHI were added thereto. The mixture was subjected to digestion reaction at 37°C for 2 hours. Then, the reaction was terminated by heat treatment at 65°C for 5 minutes, and about 0.3 μg of EcoRI—BamHI fragment of about 1.1 Kb was separated and purified by low gelling temperature agarose gel electrophoresis.

Then, 0.6 μg of the 5 Kb fragment obtained from pIN—II—A2 and 0.2 μg of the 1.1 Kb fragment obtained from pNJE—1 were subjected to ligation reaction in 20 μl of a ligase buffer solution in the presence of 0.5 units of T4DNA ligase at 4°C for 16 hours.

With the thus obtained recombinant plasmid DNA, *Escherichia coli* HB101 strain was transformed according to the conventional method to obtain transformants of $Ap^R$.

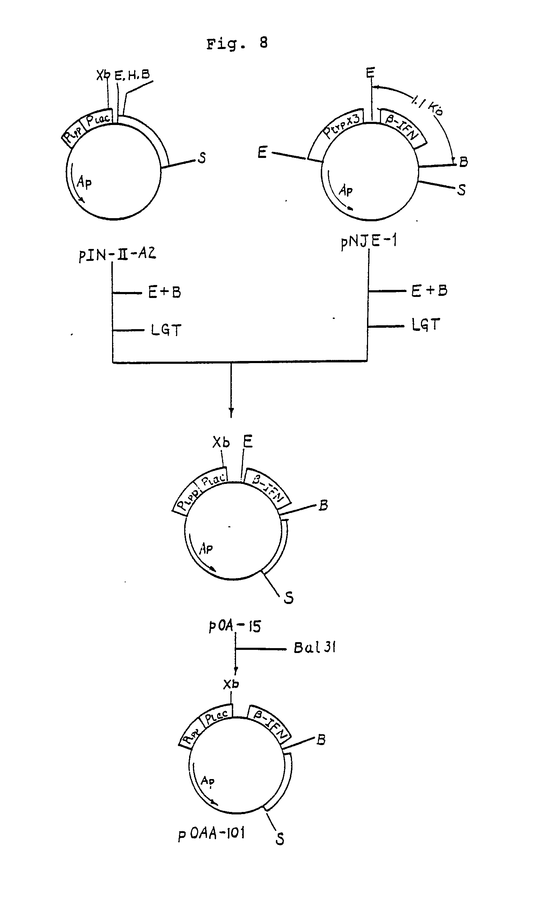

Plasmids were isolated and purified from the transformants in the same manner as above, and structural analysis of the plasmids was carried out by digesting the plasmids with restriction enzymes EcoRI, BamHI, SalI and XbaI. As a result, it was found that plasmid pOA—15 having a structure shown in Fig. 8 was constructed.

Furthermore, it was found according to the method of Maxam-Gilbert that the DNA sequence from the SD sequence to the initiation codon (ATG) of β-IFN in the plasmid pOA—15 was as follows:

$$\underline{AGAGGG}TATTAATAATG\underline{AAAGGG}$$
$$\text{SD}$$

$$\text{EcoRI}$$
$$\underline{AAGG} \; | \text{AATTCCCG} \; \boxed{\text{ATG}} \; \text{-----}$$
$$\downarrow \qquad\qquad \text{β-IFN}$$

In this case, the DNA sequence does not always have a structure suitable for polycistronic expression, but there is an SD sequence-like structure (AAGG) before the initiation codon (ATG) of β-IFN, as indicated by the dotted line, and thus an expression of β-IFN can be expected.

*Escherichia coli* strain containing pOA—15 has been deposited with the Fermentation Research Institute as *Escherichia coli* IOA—15, FERM P—6960 (FERM BP—517).

By culturing the present strain in the same manner as in Example 4, $1 \times 10^7$ units/l interferon was produced.

### Example 6

Production of β-IFN in an expression system utilizing an lpp operon;

At first, 10 μg of pOA—15 obtained in Example 5 was dissolved in 50 µl of a solution containing 12 mM CaCl₂, 12 mM MgCl₂, 600 mM NaCl, 20 mM Tris-HCl (pH 8.1) and 1 mM EDTA, and admixed with 0.5 unit of Ba131 (made by Bethesda Research Laboratories) which is an enzyme capable of cutting a double stranded DNA off the terminal. The mixture was subjected to reaction at 30°C for 30 seconds. After the reaction, 50 µl of water-saturated phenol was added to the mixture, and the mixture was thoroughly shaken and centrifuged (12,000 rpm for 5 minutes) to separate an aqueous layer. After repetition of the phenol treatment, about 45 µl of the aqueous layer was obtained, and 2.5-fold volume of ethanol was added to the aqueous layer. The mixture was left standing at −20°C for one hour, and then centrifuged (14,000 rpm for 5 minutes) to recover plasmid DNA as precipitates.

The thus obtained plasmid DNA was dissolved in 20 ml of a ligase solution, and the solution was subjected to ligation reaction in the presence of 0.5 unit of T4DNA ligase at 4°C for 18 hours.

With this reaction solution, *Escherichia coli* HB101 strain was transformed to obtain transformants of Ap$^R$.

Plasmids were isolated and purified from the transformants in the same manner as above, and structural analysis of the plasmids was carried out by digesting the plasmids with restriction enzymes BamHI, SalI, XbaI and EcoRI. As a result, it was found that plasmid pOAA—101 having a structure shown in Fig. 8 was constructed.

Furthermore, it was found according to the method of Maxam-Gilbert that the DNA sequence from the SD sequence to the initiation codon (ATG) of β-IFN in the plasmid pOAA-101 was as follows:

$$\underset{\text{SD}}{\underline{\text{AGAGGGT}}}\quad \text{ATTAATA}\quad \underset{\text{met}}{\underline{\text{ATG}}}\quad \underset{\text{lys}}{\underline{\text{AAA}}}\quad \underset{\text{gly}}{\underline{\text{GGG}}}$$

$$\underset{\text{lys}}{\underline{\text{AAG}}}\quad \underset{\text{ala}}{\underline{\text{GCC}}}\quad \underset{\text{arg}}{\underline{\text{CG}}}\,\boxed{\text{ATG}}\,\overset{\text{termination}}{\text{A ----}}$$

$$\text{β-IFN}$$

As is obvious from the above structure, the initial ATG is an initiation codon of *Escherichia coli* lipoprotein, and the peptide derived from the lipoprotein consists of 6 amino acids from met to arg, terminating at the termination codon (TGA). There is an initiation codon (ATG) of β-IFN overlapping with the termination codon, and it seems that AAGG located 9 bases apart upstream from the ATG performs the function of the SD sequence.

*Escherichia coli* strain containing pOAA—101 has been deposited with the Fermentation Research Institute as *Escherichia coli* IOAA—101, FERM P—6961 (FERM BP—518).

The present strain was cultured in the same manner as in Example 4 after induction by 20 µg/ml isopropyl-β-D-thiogalactoside, whereby 8 × 10⁷ units/l interferon was produced.

Reference Example 1

Construction of a plasmid vector pKYP—10 having a trp promoter:

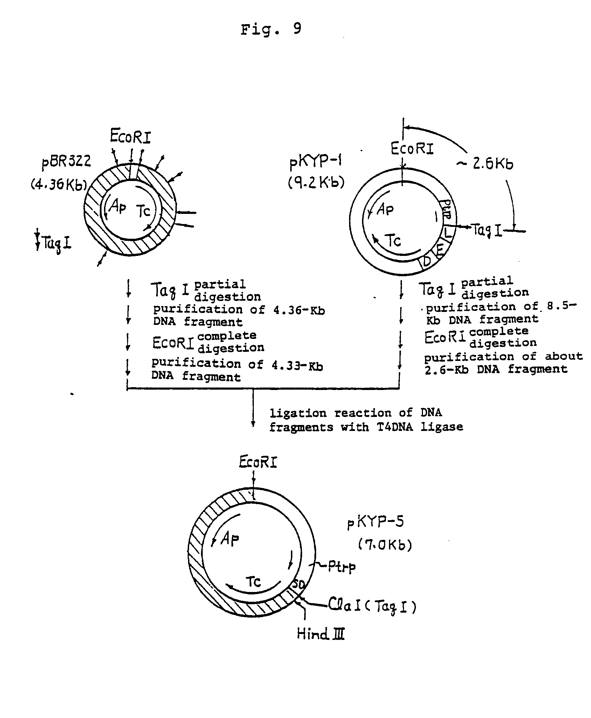

a) The plasmid pKYP—1 prepared as in Example 1 was digested with TaqI and EcoRI and the digest was subjected to purification by agarose gel electrophoresis to obtain a 2,6-Kb DNA fragment containing the tryptophan promoter and SD sequence. The 2.6-Kb DNA fragment was cloned in a known vector, pBR322, by the method as illustrated in Fig. 9. That is, 8 µg of pBR322 was digested at 45°C for 60 minutes with 2 units of TaqI in 100 µl of a reaction mixture comprising 10 mM Tris-HCl (pH 8.4), 6 mM MgCl₂, 100 mM NaCl and 6 mM 2-mercaptoethanol. After partial digestion with TaqI, the digest was subjected to low gelling temperature agarose gel electrophoresis to obtain a purified 4.36-Kb DNA fragment. About 1.5 µg of the DNA fragment was completely digested at 37°C for 3 hours with 3 units of EcoRI. About 1.0 µg of a 4.33-Kb DNA fragment was recovered by the same low gelling temperature agarose gel electrophoresis as mentioned above. Then, by the same procedure as above, 12 µg of pKYP—1 DNA was partially digested with 3 units of TaqI, the digest was purified by low gelling temperature agarose gel electrophoresis to obtain about 2 µg of a 8.5-Kb DNA fragment and the DNA was completely digested with EcoRI to obtain about 0.5 µg of a purified 2.6-Kb DNA fragment. Then, 0.4 µg of the thus obtained 4.33-Kb DNA of pBR322 and 0.25 µg of the thus obtained 2.6-Kb DNA fragment of pKYP—1 were added to 20 µl of a reaction solution consisting of 20 mM Tris-HCl (pH 7.6), 10 mM MgCl₂ and 10 mM dithiothreitol. Then, 0.5 mM ATP and 4 units of T4DNA ligase were added to the mixture and ligation reaction was carried out at 4°C for 18 hours. *Escherichia coli* C600SF8 strain was transformed with the recombinant plasmid DNA obtained as above. The plasmid in the transformant of Ap$^R$Tc$^R$ was isolated and purified. The plasmid DNA was digested with 6 restriction enyzmes, EcoRI, HindIII, ClaI, HpaI, HincII (made by Takara Shuzo Co.) and BamHI to analyze the structure of the plasmid. The plasmid was named pKYP—5.

b) Preparation of a portable promoter from the trp promoter:

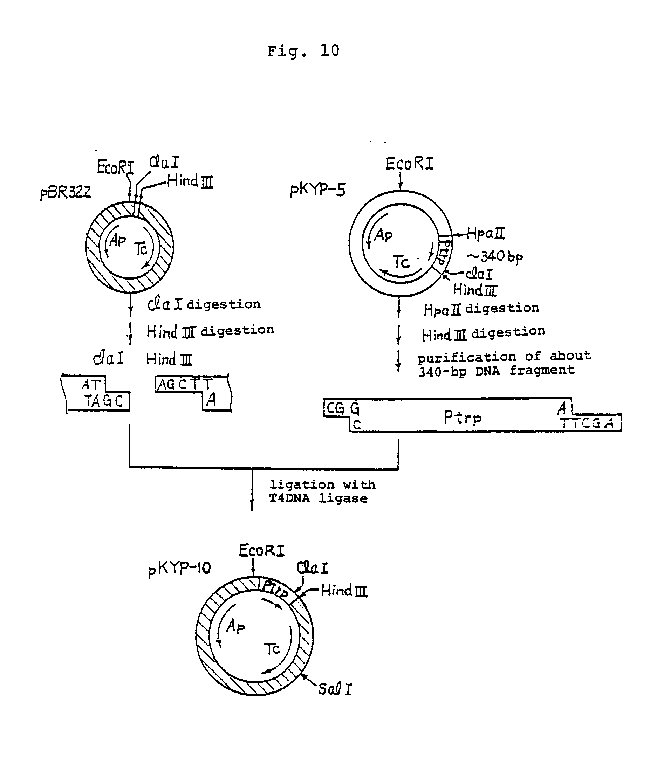

The plasmid vector pKYP—5 mentioned above is applicable as a DNA-introducing vector since it has a ClaI site and a HindIII site on the DNA of 1 to 20 base pairs downstream from the SD sequence. However, another ClaI site is present in pKYP—5 DNA besides the ClaI site immediately after the SD sequence and the fragment with the trp promoter obtained by cutting pKYP—5 DNA with EcoRI and HindIII is a little too large, i.e. 2.65 Kb. For convenience of use, pKYP—5 was improved by the process as illustrated in Fig. 10 to obtain a plasmid having a shorter DNA fragment containing a tryptophan promoter. That is, pKYP—5 DNA was digested with HpaII and HindIII and the digest was purified to obtain a DNA fragment of about 340 bp. The fragment was inserted into the

pBR322 digested with ClaI and HindIII as illustrated in Fig. 10 to obtain pKYP—10. The structure of pKYP—10 was determined by agarose gel electrophoresis after digestion with EcoRI, ClaI, HindIII and HpaI.

Reference Example 2
Construction of pKYP—100:

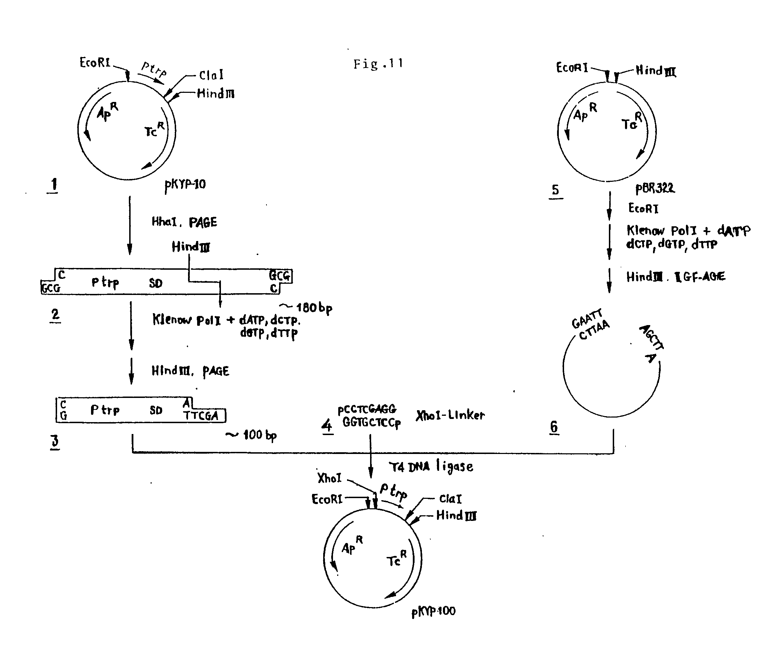

pKYP—100 which was more convenient for the construction of the plasmid vector of the present invention was constructed as illustrated in Fig. 11. As the source of DNA containing a trp promoter, plasmid pKYP—10 bearing a trp portable promoter was used.

50 µg of pKYP—10 was digested with 50 units of HhaI (made by Takara Shuzo Co.) in 100 µl of a reaction solution containing 10 mM Tris-HCl (pH 7.5), 7 mM $MgCl_2$ and 6 mM 2-mercaptoethanol at 37°C for 2 hours. After digestion with HhaI, a DNA fragment of about 180 bp containing a trp promoter was purified by 5% polyacrylamide gel electrophoresis [A. M. Maxam et al.: Proc. Natl. Acad. Sci. 74, 560 (1977), referred to as PAGE hereinafter]. In the purification step, two DNA fragments other than the desired DNA were obtained because of incomplete purification by PAGE. The three purified DNA fragments (total amount: about 4 µg) were allowed to react with 8 units of Escherichia coli DNA polymerase I· Klenow fragment in 30 µl of a reaction solution containing 50 mM Tris-HCl (pH 7.6), 7 mM $MgCl_2$, 10 mM 2-mercaptoethanol, 0.25 mM dATP, 0.25 mM dCTP, 0.25 mM dGTP, and 0.25 mM dTTP at 15°C for 2 hours. By the reaction, the 3'-protruding end formed by HhaI digestion was changed to flush end by the 3' → 5' exonuclease activity and 5' → 3' repairing synthesis activity of DNA polymerase I·Klenow fragment. Subsequently, DNA polymerase I·Klenow fragment was inactivated by heating at 72°C for 30 minutes and the NaCl concentration was adjusted to 50 mM with 1M NaCl. 8 units of HindIII was added and the mixture was allowed to react at 37°C for 2 hours. After digestion with HindIII, the DNA fragment of about 100 bp containing a trp promoter was isolated and purified by PAGE.

Separately, 5 µg of plasmid pBR322 was digested with 8 units of EcoRI in 20 µl of a reaction solution containing 10 mM Tris-HCl (pH 7.5), 50 mM NaCl, 7 mM $MgCl_2$ and 6 mM 2-mercaptoethanol at 37°C for 2 hours. After phenol and chloroform extraction and ethanol precipitation, the DNA fragment was dissoved in 20 µl of a mixture of 50 mM Tris-HCl (pH 7.6), 7 mM $MgCl_2$, 6 mM 2-mercaptoethanol, 0.25 mM dATP, 0.25 mM dCTP, 0.25 mM dGTP· and 0.25 mM dTTP. Then, 8 units of Escherichia coli DNA polymerase I· Klenow fragment was added and the mixture was allowed to react at 15°C for 2 hours. The 5'-protruding end formed by EcoRI digestion was changed to flush and by the repairing synthesis activity of DNA polymerase I· Klenow fragment. DNA polymerase I· Klenow fragment was inactivated by heating at 72°C for 30 minutes and the NaCl concentration was adjusted to 50 mM with 1

M NaCl. 8 units of HindIII was added and the mixture was allowed to react at 37°C for 2 hours. After digestion with HindIII, the larger plasmid DNA fragment of about 4.33 Kb was purified by low gelling temperature agarose gel electrophoresis.

About 50 ng of the thus obtained DNA fragment of about 100 bp containing a trp promoter, about 0.2 µg of a DNA fragment of about 4.33 Kb derived from pBR322 and 50 ng of 5'-phosphorylated XhoI linker (pCCTCGAGG, made by Collaborative Research) were ligated with 1 unit of T4DNA ligase in 20 µl of a reaction solution containing 20 mM Tris-HCl (pH 7.6), 10 mM $MgCl_2$, 10 mM dithiothreitol and 0.5 mM ATP at 4°C for 40 hours. Escherichia coli HB101 strain was transformed with the thus obtained recombinant plasmid DNA and plasmid DNAs were isolated and purified from the $Ap^R Tc^R$ transformants. These plasmid DNAs were digested with 7 restriction enzymes EcoRI, XhoI, HindIII, HaeIII, ClaI, TaqI and RsaI (made by New England Biolabs) to select the plasmid wherein the DNA fragment of about 100 bp containing a trp promoter and two XhoI linkers were cloned, which was named pKYP—100.

Reference Example 3
Insertion of the DNA coding for β-IFN into the plasmid vector pKYP—12:

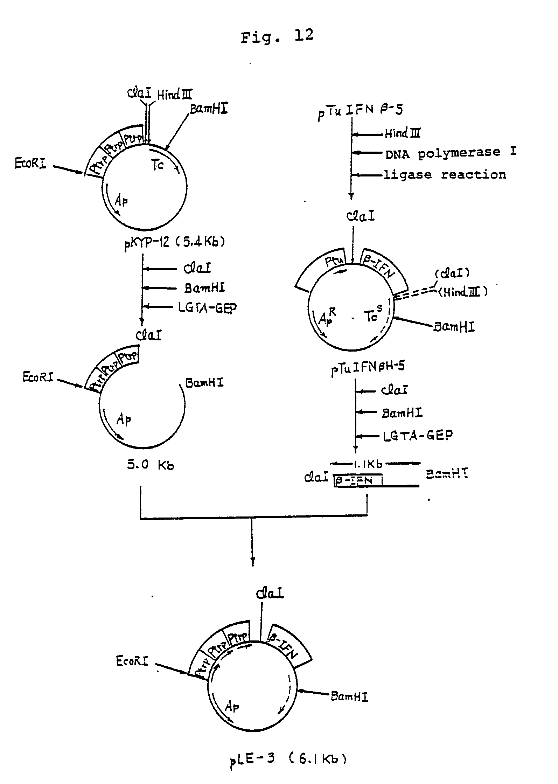

Plasmid pTuIFNβ—5 isolated from ATCC 31879 by conventional technique with digested with HindIII. The digest was treated with DNA polymerase I (made by New England Biolabs) and subjected to ligation to obtain pTuIFNβH—5 illustrated in Fig. 12. The β-IFN gene recovered from pTuIFNβH—5 was then cloned in the plasmid pKYP—12 having trp promoters. That is, 2 µg of pKYP—12 DNA was digested with 4 units of ClaI in 30 µl of a buffer solution consisting of 10 mM Tris-HCl (pH 7.5), 6 mM $MgCl_2$ and 5 mM dithiothreitol (referred to as Cla-buffer hereinafter) at 37°C for 2 hours. NaCl was added to a final concentration of 100 mM and the reaction was continued at 37°C for 2 hours. The mixture was heated at 65°C for 5 minutes to inactivate the enzyme and was then subjected to low gelling temperature agarose gel electrophoresis to obtain 1.2 µg of a purified DNA fragment of about 5 Kb containing trp promoters. Separately, 15 µg of pTuIFNβH—5 DNA was digested with ClaI and BamHI and purified by low gelling temperature agarose gel electrophoresis as described above to obtain about 1 µg of a DNA fragment (1.1 Kb) containing β-IFN gene. The two DNA fragments obtained as above and illustratged as 5 Kb and 1.1 Kb in Fig. 12 were dissolved in a mixture of 20 mM Tris-HCl (pH 7.5), 6 mM $MgCl_2$, 5 mM dithiothreitol and 500 µM ATP. 4 units of T4DNA ligase was added and the mixture was allowed to react at 4°C for 18 hours. Escherichia coli HB101 was transformed with the mixture of the thus obtained recombinants by conventional technique to obtain $Ap^R$ colonies. A plasmid was recovered from the culture of one of the colonies. The plasmid was pLE—3 illustrated in Fig. 12. The structure of pLE—3 was confirmed by agarose gel electrophoresis after the digestion of DNA with ClaI, EcoRI, HindIII and BamHI. It was found by the method of Maxam & Gilbert that the DNA sequence from the SD sequence, AAGG, to the initiation codon, ATG, in the plasmid pLE—3 was "AAGGGTATCGATG".

*Escherichia coli* containing plasmid pLE—3 has been deposited with the American Type Culture Collection (ATCC) as *Escherichia coli* ILE—3, ATCC 39010.

Reference Example 4

In this example, 2 μg of the pLE—3 DNA obtained in Reference Example 3 was dissolved in 30 μl of the Cla-buffer and 4 units of ClaI was added. The mixture was allowed to react at 37°C for 2 hours and was then heated at 65°C for 5 minutes to inactivate ClaI. Then, dGTP and dCTP were added to a concentration of 20 μM each and 6 units of *Escherichia coli* DNA polymerase I (1 μl) was added. The mixture was allowed to react at 15°C for 1 hour. Then, 5 mM dithiothreitol, 500 μM ATP and 20 units of T4DNA ligase were added and the mixture was allowed to react at 4°C for 18 hours. *Escherichia coli* HB101 was transformed using the reaction mixture in a conventional manner to obtain Ap^R colonies. A plasmid recovered from the culture of one of the colonies, named pLV—1, is illustrated in Fig. 9(a). The structure of pLV—1 was confirmed by agarose gel electrophoresis after the digestion of DNA with EcoRI, ClaI, HindIII and BamHI. It was found by the method of Maxam & Gilbert that the base sequence from the SD sequence to the initiation codon (ATG) in pLV—1 was

"AAGGGTATCGCGATG".

*Escherichia coli* containing plasmid pVL—1 has been deposited with the ATCC as *Escherichia coli* ILV—1, ATCC 39025.

Reference Example 5

Construction of recombinant plasmid pNJE—1:

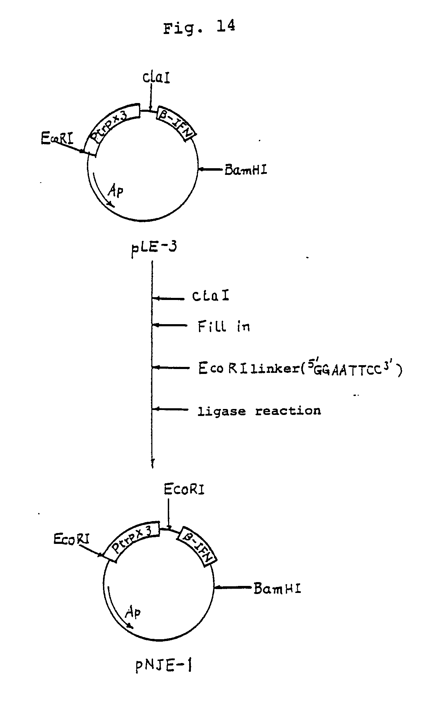

As a preliminary step of the expression of a β-IFN gene with an expression vector pIN—II—A2 having an lpp promoter, the ClaI site of plasmid pLE—3 (see Japanese Patent Application No. 213193/81. Reference Example 3) was converted to an EcoRI site by inserting an EcoRI linker as shown in Fig. 14.

First of all, 1 μg of pLE—3 was dissolved in 20 μl of a solution containing 10 mM Tris-HCl (pH 7.5), 7 mM MgCl₂, and 10 mM dithiothreitol, admixed with 2 units of ClaI and then subjected to reaction at 37°C for 2 hours. Then, the reaction was terminated by heating at 65°C for 5 minutes, and dGTP and dCTP were added thereto to a concentration of 20 μM each. Then, the mixture was further admixed with 6 units of *Escherichia coli* DNA polymerase I·Klenow fragment (1 μl) and subjected to reaction at 15°C for one hour.

Separately, 1 μg of EcoRI linker (made by Collaborative Research) was subjected to reaction in 20 μl of a solution containing 50 mM Tris-HCl

(pH 7.6), 10 mM MgCl₂ and 5 mM dithiothreitol and 1 mM EDTA in the presence of 10 units of T4 polynucleotide kinase (made by Boehringer-Mannheim) at 37°C for 30 minutes. The reaction was terminated by heating at 65°C for 10 minutes.

Then, 0.05 μg of the thus obtained phosphorylated EcoRI linker was added to the said reaction solution of pLE—3, and 500 μM ATP and 0.5 unit of T4DNA ligase were added thereto. The mixture was then subjected to ligation reaction at 4°C for 18 hours.

With this reaction solution, *Escherichia coli* HB—101 strain was transformed according to the conventional method, whereby transformants of Ap^R were obtained.

From the transformants were isolated and purified plasmids according to the said method, and one of the plasmids was digested with restriction enzymes EcoRI, BamHI and SalI to conduct structural analysis. As a result, it was found that the plasmid had the structure of plasmid pNJE—1 shown in Fig. 14. Furthermore, it was found according to the Maxama-Gilbert method that the DNA sequence from the SD sequence to the initiation codon (ATG) of β-IFN in the plasmid pNJE—1 was as follows:

<u>AAGGGTATCGGGAATTCCCGATG</u>
<u>SD</u>

*Escherichia coli* strain containing pNJE—1 has been deposited with the Fermentation Research Institute as *Escherichia coli* INJE—1, FERM P—6959 (FERM BP—516).

**Claims**

1. A process for expressing a foreign gene in a prokaryotic host characterized by using an operon having the structure which satisfies the following conditions:

a) a foreign gene is located downstream from at least one structural gene following a promoter,

b) the translational termination codon of each gene is located near the translational initiation codon of the following gene to enable the translational initiation of the following gene, and

c) a sequence requisite for the translational initiation of each gene is located upstream from the translational initiation codon of each gene.

2. A process according to claim 1, characterized in that the operon is an operon of microorganism.

3. A process according to claim 2, characterized in that the operon of microorganism is an operon of *Escherichia coli*.

4. A process according to claim 3, characterized in that the operon of *Escherichia coli* is a tryptophan operon or a lipoprotein operon.

5. A process according to claim 1, characterized in that the foreing gene is a gene of human β-interferon (hereinafter referred to as "β-IFN").

6. A plasmid wherein an operon is inserted having the structure wherein:

a) a foreign gene is located downstream from at least one structural gene following a promoter,

b) the translational termination codon of each gene is located near the translational initiation codon of the following gene to enable the translational initiation of the following gene, and

c) a sequence requisite for the translational initiation of each gene is located upstream from the translational initiation codon of each gene.

7. A plasmid according to claim 6, characterized in that the operon is an operon of microorganism.

8. A plasmid according to claim 6, characterized in that the operon of microorganism is an operon of *Escherichia coli*.

9. A plasmid according to claim 8, characterized in that the operon of *Escherichia coli* is a tryptophan operon or a lipoprotein operon.

10. A plasmid according to claim 6, characterized in that the foreign gene is a β-IFN gene.

11. A plasmid according to claim 11, characterized by the properties of efficient expression of the gene coding for β-IFN of pFN—4 contained in FERM P—6968, pOA—15 contained in FERM P—6960, or pOAA—101 contained in FERM P—6961.

12. A microorganism containing a plasmid wherein an operon is inserted, said operon having the structure wherein

a) a foreign gene is located downstream from at least one structural gene following a promoter, and

b) the translational termination codon of each gene is located near the translational initiation codon of the following gene to enable the translational initiation of the following gene

c) a sequence requisite for the translational initiation of each gene is located upstream from the translational initiation codon of each gene.

13. A microorganism according to claim 12, characterized in that the operon is an operon of microorganism.

14. A microorganism according to claim 13, characterized in that the operon of microorganism is an operon of *Escherichia coli*.

15. A microorganism according to claim 14, characterized in that the operon of *Escherichia coli* is a tryptophan operon or a lipoprotein operon.

16. A microorganism according to claim 12, characterized in that the foreign gene is a β-IFN gene.

17. A microorganism according to claim 16, wherein the plasmid is characterized by the properties of efficient expression of the gene coding for β-IFN of pFN—4, contained in FERM P—6968 pOA—15, contained in FERM P—6960 or pOAA—101 contained in FERM P—6961.

18. A microorganism according to claim 12, characterized in that the microorganism belongs to *Escherichia coli*.

19. A process for producing a substance, characterized by culturing in a culture medium a microorganism containing a plasmid wherein an operon is inserted, said operon having the structure wherein

a) a foreign gene is located downstream from at least one structural gene following a promoter, and

b) the translational termination codon of each gene is located near the translational initiation codon of the following gene to enable the translational initiation of the following gene;

c) a sequence requisite for the translational initiation of each gene is located upstream from the translational initiation codon of each gene. forming and accumulating the substance encoded by the foreign gene in the culture medium; and recovering the substance from the culture medium.

20. A process according to claim 19, characterized in that the operon is an operon of microorganism.

21. A process according to claim 20, characterized in that the operon of microorganism is an operon of *Escherichia coli*.

22. A process according to claim 21, characterized in that the operon of *Escherichia coli* is a tryptophan operon or a lipoprotein operon.

23. A process according to claim 19, characterized in that the foreign gene is a β-IFN gene.

24. A process according to claim 19, characterized in that the microorganism is a microorganism characterized by the properties of efficient expression of the gene coding for β-IFN of *Escherichia coli* IFN—4 (FERM P—6968) IOA—15 (FERM P—6960) or IOA—101 (FERM P—6961).

**Patentansprüche**

1. Verfahren zur Expression eines fremden Gens in einem prokaryontischen Wirt, dadurch gekennzeichnet, daß man ein Operon verwendet, das eine die folgenden Bedingungen erfüllende Struktur hat:

a) ein fremdes Gen befindet sich stromabwärts von mindestens einem einem Promotor folgenden strukturellen Gen,

b) das Translations-Terminationscodon von jedem Gen befindet sich in der Nähe des Translations-Initiationscodons des folgenden Gens, um die Initiation der Translation des folgenden Gens zu ermöglichen, und

c) eine Sequenz, die für die Initiation der Translation von jedem Gen erforderlich ist, befindet sich stromaufwärts vom Translations-Initiationscodon von jedem Gen.

2. Verfahren nach Anspruch 1, dadurch gekennzeichnet, daß das Operon ein Operon eines Mikroorganismus ist.

3. Verfahren nach Anspruch 2, dadurch gekennzeichnet, daß das Operon des Mikroorganismus ein Operon von Escherichia coli ist.

4. Verfahren nach Anspruch 3, dadurch gekennzeichnet, daß das Operon von Escherichia coli ein Tryptophan-Operon oder ein Lipoprotein-Operon ist.

5. Verfahren nach Anspruch 1, dadurch gekennzeichnet, daß das fremde Gen ein menschliches β-Interferon-Gen ist (im folgenden als "β-IFN" bezeichnet).

6. Plasmid, in das ein Operon mit einer Struktur eingefügt ist, bei der:

a) sich ein fremdes Gen stromabwärts von mindestens einem einem Promotor folgenden strukturellen Gen befindet,

b) sich das Translations-Terminationscodon von jedem Gen in der Nähe des Translations-Initiationscodons des folgenden Gens befindet, um die Initiation der Translation des folgenden Gens zu ermöglichen, und

c) sich eine Sequenz, die für die Initiation der Translation von jedem Gen erforderlich ist, stromaufwärts vom Translations-Initiationscodon von jedem Gen befindet.

7. Plasmid nach Anspruch 6, dadurch gekennzeichnet, daß das Operon ein Operon eines Mikroorganismus ist.

8. Plasmid nach Anspruch 6, dadurch gekennzeichnet, daß das Operon des Mikroorganismus ein Operon von Escherichia coli ist.

9. Plasmid nach Anspruch 8, dadurch gekennzeichnet, daß das Operon von Escherichia coli ein Tryptophan-Operon oder ein Lipoprotein-Operon ist.

10. Plasmid nach Anspruch 6, dadurch gekennzeichnet, daß das fremde Gen ein β-IFN-Gen ist.

11. Plasmid nach Anspruch 10, dadurch gekennzeichnet durch die Eigenschaften effizienter Expression des für β-IFN codierenden Gens von pFN—4, enthalten in FERM P—6968, pOA—15, enthalten in FERM P—6960, oder pOAA—101, enthalten in FERM P—6961.

12. Mikroorganismus, der ein Plasmid enthält, in das ein Operon mit einer Struktur eingefügt ist, bei der:

a) sich ein fremdes Gen stromabwärts von mindestens einem einem Promotor folgenden strukturellen Gen befindet, und

b) sich das Translations-Terminationscodon von jedem Gen in der Nähe des Translations-Initiationscodons des folgenden Gens befindet, um die Initiation der Translation des folgenden Gens zu ermöglichen,

c) sich eine Sequenz, die für die Initiation der Translation von jedem Gen erforderlich ist, stromaufwärts vom Translations-Initiationscodon von jedem Gen befindet.

13. Mikroorganismus nach Anspruch 12, dadurch gekennzeichnet, daß das Operon ein Operon eines Mikroorganismus ist.

14. Mikroorganismus nach Anspruch 13, dadurch gekennzeichnet, daß das Operon des Mikroorganismus ein Operon von Escherichia coli ist.

15. Mikroorganismus nach Anspruch 14, dadurch gekennzeichnet, daß das Operon von Escherichia coli ein Tryptophan-Operon oder ein Lipoprotein-Operon ist.

16. Mikroorganismus nach Anspruch 12, dadurch gekennzeichnet, daß das fremde Gen ein β-IFN-Gen ist.

17. Mikroorganismus nach Anspruch 16, wobei das Plasmid durch die Eigenschaften effizienter Expression des für β-IFN codierenden Gens von pFN—4, enthalten in FERM P—6968, pOA—15, enthalten in FERM P—6960, oder pOAA—101, enthalten in FERM P—6961, gekennzeichnet ist.

18. Mikroorganismus nach Anspruch 12, dadurch gekennzeichnet, daß der Mikroorganismus zu Escherichia coli gehört.

19. Verfahren zur Herstellung eines Stoffes, gekennzeichnet durch Züchtung eines Mikroorganismus in einem Kulturmedium, der ein Plasmid enthält, in das ein Operon mit einer Struktur eingefügt ist, bei der:

a) sich ein fremdes Gen stromabwärts von mindestens einem einem Promotor folgenden strukturellen Gen befindet, und

b) sich das Translations-Terminationscodon von jedem Gen in der Nähe des Translations-Initiationscodons des folgenden Gens befindet, um die Initiation der Translation des folgenden Gens zu ermöglichen,

c) sich eine Sequenz, die für die Initiation der Translation von jedem Gen erforderlich ist, stromaufwärts vom Translations-Initiationscodon von jedem Gen befindet,
Bildung und Ansammlung des durch das fremde Gen codierten Stoffes im Kulturmedium und Gewinnung des Stoffes aus dem Kulturmedium.

20. Verfahren nach Anspruch 19, dadurch gekennzeichnet, daß das Operon ein Operon eines Mikroorganismus ist.

21. Verfahren nach Anspruch 20, dadurch gekennzeichnet, daß das Operon des Mikroorganismus ein Operon von Escherichia coli ist.

22. Verfahren nach Anspruch 21, dadurch gekennzeichnet, daß das Operon von Escherichia coli ein Tryptophoran-Operon oder ein Lipoprotein-Operon ist.

23. Verfahren nach Anspruch 19, dadurch gekennzeichnet, daß das fremde Gen ein β-IFN-Gen ist.

24. Verfahren nach Anspruch 19, dadurch gekennzeichnet, daß der Mikroorganismus ein Mikroorganismus, gekennzeichnet durch die Eigenschaften effizienter Expression des für β-IFN codierenden Gens von Escherichia coli IFN—4 (FERM P—6968), IOA—15 (FERM P—6960) oder IOA—101 (FERM P—6961) ist.

## Revendications

1. Procédés d'expression d'un gène étranger dans un hôte procaryote, caractérisé en ce qu'on utilise un opéron ayant une structure qui satisfait les conditions suivantes:

a) le gène étranger est situé en aval d'au moins un gène de structure, à la suite d'un promoteur,

b) le codon de terminaison de traduction de chaque gène est situé près du codon d'initiation de traduction du gène suivant, pour permettre l'initiation de la traduction du gène suivant, et

c) un séquence nécessaire pour l'initiation de la traduction de chaque gène est située en amont du codon d'initiation de traduction de chaque gène.

2. Procédé conforme à la revendication 1, caractérisé en ce que l'opéron est un opéron de microorganisme.

3. Procédé conforme à la revendication 2, caractérisé en ce que l'opéron de microorganisme est un opéron d'*Escherichia coli*.

4. Procédé conforme à la revendication 3, caractérisé en ce que l'opéron *d'Escherichia coli* est un opéron tryptophane ou un opéron lipoprotéine.

5. Procédé conforme à la revendication 1, caractérisé en ce que le gène étranger est un gène d'interféron β humain (désigné ci-après par "IFN-β").

6. Plasmide dans lequel est inséré un opéron, présentant une structure dans laquelle:

a) un gène étranger est situé en aval d'au moins un gène de structure, à la suite d'un promoteur,

b) le codon de terminaison de traduction de chaque gène est situé près du codon d'initiation de traduction du gène suivant, pour permettre l'initiation de la traduction du gène suivant, et

c) une séquence nécessaire pour l'initiation de la traduction de chaque gène est située en amont du codon d'initiation de traduction de chaque gène.

7. Plasmide conforme à la revendication 6, caractérisé en ce que l'opéron est un opéron de microorganisme.

8. Plasmide conforme à la revendication 6, caractérisé en ce que l'opéron de microorganisme est un opéron *d'Escherichia coli*.

9. Plasmide conforme à la revendication 8, caractérisé en ce que l'opéron *d'Escherichia coli* est un opéron tryptophane ou un opéron lipoprotéine.

10. Plasmide conforme à la revendication 6, caractérisé en ce que le gène étranger est un gène d'IFN-β.

11. Plasmide conforme à la revendication 10, caractérisé par les propriétés d'expression efficace du gène codant pour l'IFN-β de pFN-4 contenu dans FERM P—6968, pOA—15 contenu dans FERM P—6960, ou pOAA—101 contenu dans FERM P—6961.

12. Microorganisme contenant un plasmide dans lequel est inséré un opéron, ledit opéron ayant une structure dans laquelle

a) un gène étranger est situé en aval d'au moins un gène de structure, à la suite d'un promoteur, et

b) le codon de terminaison de traduction de chaque gène est situé près du codon d'initiation de traduction du gène suivant, pour permettre l'initiation de la traduction du gène suivant, et

c) une séquence nécessaire pour l'initiation de la traduction de chaque gène est située en amont du codon d'initiation de traduction de chaque gène.

13. Microorganisme conforme à la revendication 12, caractérisé en ce que l'opéron est un opéron de microorganisme.

14. Microorganisme conforme à la revendication 13, caractérisé en ce que l'opéron de microorganisme est un opéron *d'Escherichia coli.*

15. Microorganisme conforme à la revendication 14, caractérisé en ce que l'opéron *d'Escherichia coli* est un opéron tryptophane ou un opéron lipoprotéine.

16. Microorganisme conforme à la revendication 12, caractérisé en ce que le gène étranger est un gène d'IFN-β.

17. Microorganisme conforme à la revendication 16, dans lequel le plasmide est caractérisé par les propriétés d'expression efficace du gène codant pour l'IFN-β de pFN—4 contenu dans FERM P—6968, de pOA—15 contenu dans FERM P—6960, ou de pOAA—101 contenu dans FERM P—6961.

18. Microorganisme conforme à la revendication 12, caractérisé en ce que le microorganisme appartient au genre *Escherichia coli.*

19. Procédé de production d'une substance, caractérisé par la culture, dans un milieu de culture, d'un microorganisme contenant un plasmide dans lequel est inséré un opéron, ledit opéron présentant une structure dans laquelle

a) un gène étranger est situé en aval d'au moins un gène de structure, à la suite d'un promoteur,

b) le codon de terminaison de traduction de chaque gène est situé près du codon d'initiation de traduction du gène suivant, pour permettre l'initiation de la traduction du gène suivant, et

c) une séquence nécesssaire pour l'initiation de la traduction de chaque gène est située en amont du codon d'initiation de traduction de chaque gène,

la formation et l'accumulation de la substance codée par le gène étranger dans le milieu de culture, et la récupération de cette substance à partir du milieu de culture.

20. Procédé conforme à la revendication 19, caractérisé en ce que l'opéron est un opéron de microorganisme.

21. Procédé conforme à la revendication 20, caractérisé en ce que l'opéron de microorganisme est un opéron *d'Escherichia coli*.

22. Procédé conforme à la revendication 21, caractérisé en ce que l'opéron *d'Escherichia coli* est un opéron tryptophane ou on opéron lipoprotéine.

23. Procédé conforme à la revendication 19, caractérisé en ce que le gène étranger est un gène d'IFN-β.

24. Procédé conforme à la revendication 19, caractérisé en ce que le microorganisme est un microorganisme caractérisé par les propriétés d'expression efficace du gène codant pour l'IFN-β *d'Escherichia coli* IFN—4 (FERM P—6968), IOA—15 (FERM P—6960) ou IOA—101 (FERM P—6961).

Fig. 1

Fig.2

EP 0 126 338 B1

Fig.3

pKYP-8

pKYP-9

3

Fig.4

Fig. 5

pKYP-108

GAATTCCTCGAGGCCTCGAGGCCGACATCATAACGGTTCTGGCAAATATTCTGAAATGAGCTGTTGACAA
EcoRI  XhoI    XhoI

                                                      Met Lys Ala Ile
TTAATCATCGAACTAGTTAACTAGTACGCAAGTTCACGTAAAAAGGGTATCGACA ATG AAA GCA ATT
                                                            trpL

Phe Val Leu Lys Gly Trp Trp Arg Thr Ser Umb
TTC GTA CTG AAA GGT TGG TGG CGC ACT TCC TGA AACGGGCAGTGTATTCACCATGCGTA

                                                      Met
AAGCAATCAGATACCCAGCCCGCCTAATGAGCGGGCTTTTTTTTTGAACAAAATTAGAGAATAACA ATG
                                                            trpE
Gln Thr Gln Lys Pro Thr Leu
CAA ACA CAA AAA CCG ACT CTC GATAAGCTT
                           HindIII

pKYP-109

GAATTCCTCGAGGCCTCGAGGCCGACATCATAACGGTTCTGGCAAATATTCTGAAATGAGCTGTTGACAA
EcoRI  XhoI    XhoI

                                                      Met Lys Ala Ile
TTAATCATCGAACTAGTTAACTAGTACGCAAGTTCACGTAAAAAGGGTATCGACA ATG AAA GCA ATT
                                                            trpL
Phe Val Leu Lys Gly Trp Trp Arg Thr Ser Umb
TTC GTA CTG AAA GGT TGG TGG CGC ACT TCC TGA AACGGGCAGTGTATTCACCATGCGTA

                                                      Met
AAGCAATCAGATACCCAGCCCGCCTAATGAGCGGGCTTTTTTTTTGAACAAAATTAGAGAATAACA ATG
                                                            trpE
Gln Thr Gln Lys Pro Thr Leu Gln Leu Leu Thr Cys Gln Gly Ala Tyr Arg Asp
CAA ACA CAA AAA CCG ACT CTC GAA CTG CTA ACC TGC GAA GGC GCT TAT CGC GAC
                                                                     NruI
Asn Pro Thr Ala Leu Phe His Gln Leu Cys Gly Asp Arg Pro Ala Thr Leu Leu
AAT CCC ACC GCG CTT TTT CAC CAG TTG TGT GGC GAT CGT CCG GCA ACG CTG CTG

Leu Gln Ser Ala Asp Ile
CTG GAA TCC GCA GAT ATC GATAAGCTT
                         ClaI   HindIII

5

Fig. 6

Fig. 7

```
                  trpE gene
                    Met Gln Thr Gln Lys Pro Thr Leu Gln Leu Leu Thr
AATTAGAGAATAACA ATG CAA ACA CAA AAA CCG ACT CTC GAA CTG CTA ACC
      SD

Cys Gln Gly Ala Tyr Arg Thr Ser Thr Gln Val His Val Lys Arg Val
TGC GAA GGC GCT TAT CGA ACT AGT ACG CAA GTT CAC GTA AAA AGG GTA
                                                          SD

Ser Arg Stop
TCG CG ATG AGC TAC AAC TTG CTT GGA TTC CTA
 NruI  Met Ser Tyr Asn Leu Leu Gly Phe Leu

         β-IFN gene
```

Fig. 8

Fig. 9

Fig. 10

Fig.11

# EP 0 126 338 B1

## Fig. 12

Fig. 13

Fig. 14

pLE-3

claI

Fill in

EcoRI linker($^5'$GGAATTCC$^{3'}$)

ligase reaction

pNJE-1